# EUROPÄISCHE PATENTSCHRIFT

(11) **EP 1 721 139 B1**
(45) Veröffentlichungstag und Bekanntmachung des Hinweises auf die Patenterteilung: **13.04.2016**
(21) Anmeldenummer: 05707556.6
(22) Anmeldetag: 22.02.2005
(51) Int. Cl.: G01N 1/00, G01N 33/487, G01N 1/14, G01N 21/31, A01C 23/00, G01N 33/24, G01N 35/10, G01N 1/20, G01N 21/05

(54) **FAHRZEUG MIT ANORDNUNG ZUR SPEKTROSKOPISCHEN BESTIMMUNG DER BESTANDTEILE UND KONZENTRATIONEN PUMPFÄHIGER ORGANISCHER VERBINDUNGEN UND VERFAHREN**
VEHICLE WITH SYSTEM FOR THE SPECTROSCOPIC DETERMINATION OF THE COMPONENTS AND CONCENTRATIONS OF PUMPABLE ORGANIC COMPOUNDS AND METHOD
VEHICULE AVEC ENSEMBLE DE DETERMINATION SPECTROSCOPIQUE DES CONSTITUANTS ET DES CONCENTRATIONS DE COMPOSES ORGANIQUES APTES AU POMPAGE ET PROCEDE

(30) Priorität: 27.02.2004 DE 102004010217
(43) Veröffentlichungstag der Anmeldung: 15.11.2006
(73) Patentinhaber: Carl Zeiss Spectroscopy GmbH, 07745 Jena (DE)
(72) Erfinder: RODE, Michael, 07743 Jena (DE); ANDREE, Helga, 24105 Kiel (DE); MALLEY, Diane, F., Nanaimo, British Columbia V9V 1L6 (CA)
(74) Vertreter: Müller, Silke
(86) Internationale Anmeldenummer: PCT/EP2005/001799
(87) Internationale Veröffentlichungsnummer: WO 2005/083386

(56) Entgegenhaltungen:
- US-A- 5 420 432
- US-B1- 6 263 725
- US-B1- 6 657 718
- PATENT ABSTRACTS OF JAPAN Bd. 1999, Nr. 12, 29. Oktober 1999 (1999-10-29) & JP 11 196693 A (MITSUBISHI AGRICULT MACH CO LTD), 27. Juli 1999 (1999-07-27)
- DATABASE WPI Section Ch, Week 200224 Derwent Publications Ltd., London, GB; Class C07, AN 2002-186795 XP002328079 -& NL 1 015 440 C2 (VMA VLASTUIN MEST APPLICATIES BV) 17. Dezember 2001 (2001-12-17)
- PATENT ABSTRACTS OF JAPAN Bd. 006, Nr. 038 (P-105), 9. März 1982 (1982-03-09) & JP 56 155850 A (TOSHIBA CORP), 2. Dezember 1981 (1981-12-02)

## Beschreibung

Die vorliegende Erfindung betrifft ein schnelles und zerstörungsfreies Messverfahren zur Bestimmung der Inhaltstoffe fester, flüssiger und/oder suspensiver organischer im Fluss befindlicher Verbindungen. Die vorgeschlagene Lösung ist geeignet auf eine Vielzahl relevanter Parameter kalibriert zu werden und kann in allen Bereichen, insbesondere auch mobil eingesetzt werden.

Die in der DE 100 16 023 C2 beschriebene Durchfluss-Messküvette ist für die kombinierte Benutzung der Spektroskopie und der Polarimetrie zur gleichzeitigen Bestimmung mehrerer Messgrößen bei physikalisch-chemischen und biotechnischen Prozessen geeignet. Insbesondere lassen sich gelöste Substanzen in durchfließenden Medien kontinuierlich und ohne zeitliche Verzögerung detektieren und quantitativ erfassen. Für die spektroskopische Messung sind Wellenlängen vom UV-Bereich bis in den NIR-Bereich nutzbar. Die Messstrecke befindet sich zwischen zwei, quer zur Flussrichtung in die Messküvette ragende Stäbe aus Glas o. ä.. In Abhängigkeit von der zu untersuchenden Substanz muss die Messstrecke durch verschieben der Stäbe variiert werden, da nur Messungen in Transmission möglich sind. Die Verwendung der Durchfluss-Messküvette für den automatisierten, mobilen Einsatz wird dadurch allerdings erschwert bzw. verhindert.

Die Bestimmung der Inhaltsstoffe von Gülle ist beispielsweise unter dem Aspekt eines präzisen Einsatzes als organischen Dünger von besonderer Bedeutung. Die hier geltenden Vorgaben die maximale Ausbringmenge betreffend konnten bisher nur sehr schwer eingehalten werden, da eine exakte Bestimmung der Bestandteile und Konzentrationen zum Zeitpunkt der Ausbringung nicht möglich war. Eine Kontrolle der Einhaltung der Vorgaben war nur durch Bodenanalysen vor bzw. nach der Ausbringung möglich.

Nach dem bekannten Stand der Technik erfolgt die Bestimmung der Inhaltsstoffe nach der Probenentnahme durch anschließender nasschemische Messungen im Labor. Dieses Verfahren ist zeitaufwendig und erfordert eine erhebliche Probenvorbereitung. Die Anzahl der Proben ist limitiert, der Einsatz der Analysedaten zur zeitnahen Steuerung/Regelung von Prozessen ist nur begrenzt bis überhaupt nicht möglich. Erfolgt die Messung in Durchflusszellen mit herkömmlichen Sensoren, so können jedoch nur die Inhaltsstoffe erfasst werden, die über die jeweiligen Sensoren selektiv messbar sind.

Von der Firma PDK Projects, Inc. wurde in einem Projekt ("Analysis of Nutrients in Hog Manure by Field-portable Near-infrared Spectroscopy", Juli 2001) die Verwendung der NIR-Spektroskopie zur Bestimmung der Bestandteile und Konzentrationen organischer Reststoffe nachgewiesen. Die Ergebnisse dieses Projekts bezogen sich allerdings nur auf die Verwendung spektroskopischer Messgeräte unter Laboreinsatz. Die zu bestimmenden Proben wurden dabei auf den spektroskopischen Messkopf gestellt. Eine kompakte Lösung für einen möglichen mobilen Einsatz auf einem Fahrzeug zum Ausbringen von Gülle war nicht Ziel des Projekts.

Der vorliegenden Erfindung liegt die Aufgabe zugrunde ein schnelles und zerstörungsfreies Messverfahren sowie eine entsprechende Anordnung zur Bestimmung der Inhaltsstoffe fester, flüssiger und/oder suspensiver organischer Reststoffe zu entwickeln. Die Lösung soll für den mobilen Einsatz geeignet sein und eine hohe, kontinuierliche Messdatendichte realisieren.

Erfingdungsgemäß wird die Aufgabe durch die Merkmale der unabhängigen Ansprüche gelöst. Bevorzugte Weiterbildungen und Ausgestaltungen sind Gegenstand der abhängigen Ansprüche.

Die vorgeschlagene technische Lösung liefert repräsentative Messwerte der Inhaltsstoffe von inhomogenem Material im Durchflussverfahren, wobei eine direkte Messung durch Lichtabsorption bzw. -transmission erfolgt. Das Transflexionsprinzip ermöglicht Messungen sowohl an stark absorbierenden als auch an transmissiven Proben, d. h. auch von trüben Suspensionen, und erfasst nicht nur die flüssigen, sondern auch die festen Bestandteile der Probe.

Durch den kompakten Aufbau ist die Lösung insbesondere auch für den mobilen Einsatz zur Bestimmung der Inhaltsstoffe fester, flüssiger und/oder suspensiver organischer Verbindungen geeignet.

Beispielsweise ist die Lösung zur Bestimmung der Inhaltsstoffe von Gülle als organischer Dünger einsetzbar. Durch eine Vorort-Bestimmung, insbesondere auch während des Ausbringens, kann der Dünger, in Abhängigkeit der direkt ermittelten Inhaltstoffe gezielt dosiert werden.

Prinzipiell lässt sich die vorgeschlagene technische Lösung jedoch auf beliebige Anwendungen mit Suspensionen bzw. pumpfähige, homogene und inhomogene Materialien übertragen. Insbesondere ist eine Anwendung in der Lebensmittelindustrie, der Abwasserkontrolle und der Prozessüberwachung, hierbei z. B. in der Fleischverarbeitung und der Produktion von Biogas, denkbar.

Die Erfindung wird nachfolgend anhand eines Ausführungsbeispieles näher beschrieben. Dazu zeigt
- Figur 1:: eine Prinzipskizze einer Anordnung für den Laboreinsatz und
- Figur 2:: eine für den mobilen Einsatz auf einem Fahrzeug angepasste Anordnung.

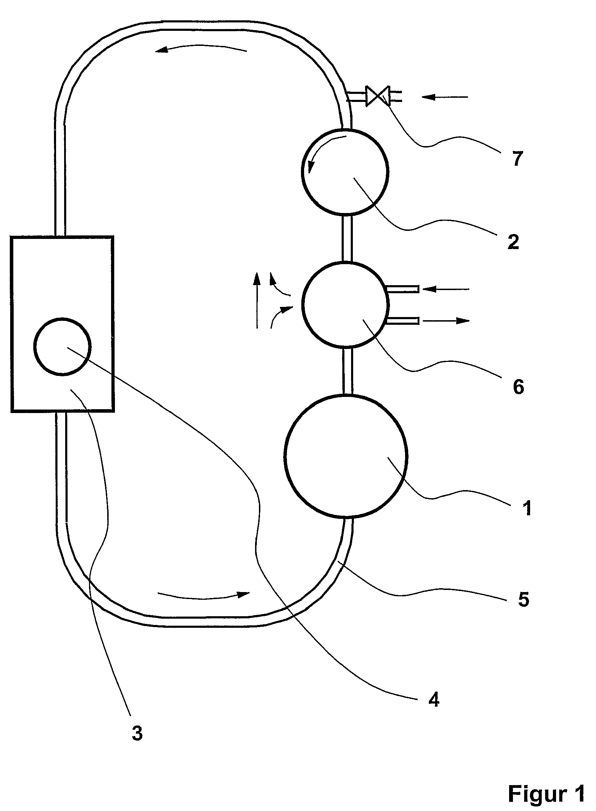

**Figur 1** zeigt eine Anordnung zur spektroskopischen Bestimmung der Bestandteile und Konzentrationen pumpfähiger organischer Verbindungen. Die Anordnung besteht aus einem Probenbehälter **1,** einer Pumpe **2** und einer Messzelle **3,** die mit einem spektroskopischen Messkopf **4** eine Einheit bildet. Die Messzelle **3** ist mit der Pumpe **2,** die zur Variation der Fließgeschwindigkeit regelbar ist, und dem Probenbehälter **1** über eine Rohrleitung **5** verbunden. Der spektroskopische Messkopf **4** und die regelbare Pumpe **2** weisen zu einer (nicht dargestellten) Steuer- und Auswerteeinheit elektrische Verbindungen auf.

Die Messzelle **3** ist vorzugsweise so ausgebildet, dass die Probe zwischen zwei gegenüberliegend, senkrecht zur Flussrichtung in die Messzelle integrierten Fenstern, durchfließt. Beim Durchfluss der Probe durch die Messzelle **3** muss gewährleistet sein, dass keine Sedimentation, Schaumbildung und Verstopfung entstehen. Die optimale Fließgeschwindigkeit variiert dabei je nach Probentyp in Abhängigkeit von Trockenmassegehalt, Partikelgröße, Partikelstruktur und Thixotropie.

Um die Messzelle **3** von Resten der gemessenen Probe zu säubern und diese für die nächste Probe vorzubereiten ist ein Mehrwegeventil **6** vorgesehen, welches Verbindungen zu einem Wasserbehälter und/oder ein Behälter mit Reinigungsflüssigkeit herstellt. Das Mehrwegeventil **6** verfügt über einen Stellantrieb, der mit der Steuer- und Auswerteeinheit verbunden ist. Über dieses Mehrwegeventil **6** kann die Messzelle zusätzlich mit Behältern verbunden werden, in denen Testflüssigkeiten zur Selbstkalibrierung der Messanordnung enthalten sind.

Sowohl die verwendete Reinigungsflüssigkeit als auch Spülwasser kann nach dem Durchspülen der Messzelle **3** über das Mehrwegeventil **6** ausgestoßen werden. Vorteilhaft ist dabei ein mehrmaliges Durchspülen der Messzelle **3.**

Der Verschmutzungsgrad und eine eventuell notwendige Reinigung der Messzelle **3** kann durch eine spektroskopische Messung der Messzelle **3** ohne Probe festgestellt werden.

Weiterhin kann die Anordnung über eine Vorrichtung zur Trocknung der Messzelle **3** verfügen, die ebenfalls mit der Steuer- und Auswerteeinheit verbunden ist. Die Trocknung erfolgt dabei beispielsweise durch Belüftung, indem über ein Ventil **7** Luft durch die der Messzelle **3** gedrückt wird. Auch die Luft kann nach dem Durchströmen der Messzelle **3** über das Mehrwegeventil **6** ausgestoßen werden.

Da von einem Temperatureinfluss auf die Messergebnisse auszugehen ist, wird eine Vorrichtung zur Temperierung der Probe, vorzugsweise in oder vor der Messzelle **3,** vorgesehen.

In einer vorteilhaften Ausgestaltung wird die Anordnung auf einem Fahrzeug, insbesondere zum Ausbringen pumpfähiger organischer Reststoffe montiert sein.

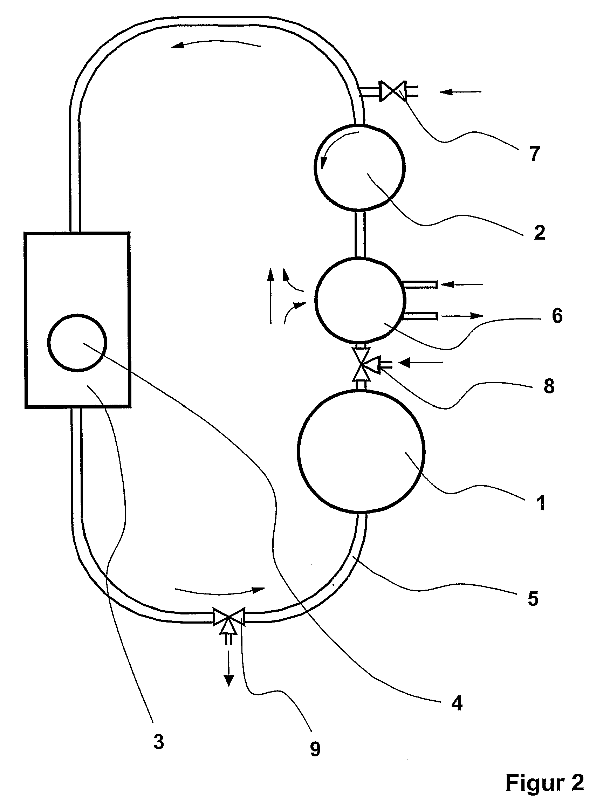

**Figur 2** zeigt dazu eine für den mobilen Einsatz auf einem Fahrzeug angepasste Anordnung. Dazu sind in der Rohrleitung **5** zwei zusätzliche Dreiwegeventile **8** und **9** angeordnet. Zur Bestimmung der Bestandteile und Konzentrationen während des Ausbringens wird über das Dreiwegeventile **8** eine Probe aus der Auslassleitung des Tankes entnommen, durch die Messzelle **3** gepumpt und über das Dreiwegeventile **9** zurück in die Auslassleitung des Tankes geleitet. Die Dreiwegeventile **8** und **9** verfügen dabei vorzugsweise ebenfalls über Stellantriebe, der mit der Steuer- und Auswerteeinheit verbunden sind. Der Probenbehälter 1 kann dabei in der Anordnung verbleiben, damit für den Spül- und/oder Trockenvorgang ein geschlossener Kreislauf vorhanden ist.

Die Durchflussmenge eines am Tank vorhandenen Auslassventils kann von der Steuer- und Auswerteeinheit, in Auswertung der ermittelten Bestandteile und Konzentrationen der pumpfähigen organischen Reststoffe geregelt werden. Zusätzlich zu den ermittelten Bestandteilen und Konzentrationen in der Probe enthaltener Stoffe, können von der Steuer- und Auswerteeinheit zuvor bestimmte Bodenwerte sowie die Momentangeschwindigkeit des Fahrzeuges berücksichtigt werden um ein geeignetes Steuersignal zur Regelung des Durchflusses eines Auslassventils beim Ausbringen pumpfähiger organischer Reststoffe zu generieren. Dadurch kann eine dem Bedarf des Bodens entsprechende Düngung vorgenommen werden.

Da die Partikelgröße organischer Reststoffe sehr stark variieren und unter Umständen zu Verstopfungen führen kann, ist es zweckmäßig vor der Pumpe **2** ein Schneidwerk u./o. Sieb anzuordnen. Zu große Partikel können dadurch zerkleinert werden, ohne dass die Zusammensetzung der Probe verändert wird.

Die Bestimmung der Inhaltsstoffe von Gülle ist beispielsweise unter dem Aspekt eines präzisen Einsatzes als organischen Dünger von besonderer Bedeutung. Für die Einhaltung der hier geltenden Vorgaben ist es sinnvoll, dass die ausgebrachte Menge an organischen Reststoffe bezogen auf die Bestandteile und Konzentrationen der einzelnen Inhaltstoffe protokolliert werden. Ein Nachweis der ausgebrachte Menge an organischen Reststoffen ist dadurch ohne weiteres möglich.

Bei dem erfindungsgemäßen Verfahren zur spektroskopischen Bestimmung der Bestandteile und Konzentrationen pumpfähiger organischer Reststoffe wird die in einem Probenbehälter 1 enthaltene Probe von einer Pumpe **2** durch eine Messzelle **3,** die mit einem spektroskopischen Messkopf **4** eine Einheit bildet, gefördert. Der Messkopf **4** nimmt eine spektroskopische Messung der durch die Messzelle **3** fließenden Probe in Transmission und/oder Reflexion vor und leitet die Messergebnisse zur weiteren Verarbeitung an eine Steuer- und Auswerteeinheit weiter. Von dieser werden anhand hinterlegter spezifischer Kalibrierungen Bestandteile und Konzentrationen in der Probe enthaltener Stoffe ermittelt. Dazu sind temperatur-, durchfluss- und probenabhängige spezifische Kalibrierungen erforderlich.

Die Messzelle **3** ist vorzugsweise so ausgebildet, dass die Probe zwischen zwei gegenüberliegend, senkrecht zur Flussrichtung in die Messzelle integrierten Fenstern, durchfließt. Zur Einstellung der für die spektroskopische Messung erforderliche Fließgeschwindigkeit der Probe ist die Pumpe **2** regelbar. Dadurch kann gewährleistet werden, dass es beim Durchfluss der Probe durch die Messzelle **3** keine Sedimentation, Schaumbildung und Verstopfung entstehen. Die optimale Fließgeschwindigkeit variiert dabei je nach Probentyp in Abhängigkeit von Trockenmassegehalt, Partikelgröße, Partikelstruktur und Thixotropie.

Zur Gewährleistung unverfälschter Messwerte wird die Messzelle **3** nach erfolgter Messung einer Probe gesäubert. Dazu wird ein vorhandener Wasserbehälter über ein Mehrwegeventil 6 mit der Messzelle **3** verbunden wird, um Resten der gemessenen Probe zu entfernen. Es ist aber auch möglich das ein weiterer Behälter mit Reinigungsflüssigkeit vorhanden ist, der über das Mehrwegeventil **6** mit der Messzelle **3** verbunden wird. Nach erfolgter Säuberung wird die Messzelle **3** mit Wasser gespült. Unter Umständen ist nach der Säuberung und Spülung eine Trocknung der Messzelle **3** erforderlich. Die Trocknung erfolgt dabei durch Belüftung, indem über ein Ventil **7** Luft durch die Messzelle **3** gedrückt wird.

Zur Vermeidung temperaturbeeinflusster Messergebnisse können die Proben vor dem Messvorgang durch eine Vorrichtung temperiert werden. Dies sollte vorzugsweise in oder vor der Messzelle **3** erfolgen.

In einer vorteilhaften Ausgestaltung kann das beschriebene Verfahren auf einem Fahrzeug, insbesondere zum Ausbringen pumpfähiger organischer Reststoffe angewendet werden. Von der Steuer- und Auswerteeinheit wird anhand der ermittelten Bestandteilen und Konzentrationen in der Probe enthaltener Stoffe ein zusätzliches Steuersignal zur Regelung des Durchflusses eines Auslassventils beim Ausbringen pumpfähiger organischer Reststoffe generiert.

Eine besonders vorteilhafte Lösung ergibt sich, wenn von der Steuer- und Auswerteeinheit zusätzlich zu den ermittelten Bestandteilen und Konzentrationen in der Probe enthaltener Stoffe, zuvor bestimmte Bodenwerte sowie die Momentangeschwindigkeit des Fahrzeuges berücksichtigt werden um ein Steuersignal zur Regelung des Durchflusses eines Auslassventils beim Ausbringen pumpfähiger organischer Reststoffe zu generieren.

Dadurch ist eine noch gezieltere Ausbringung von Gülle als organischer Dünger realisierbar. Durch die zuvor bestimmte Bodenwerte ist insbesondere während des Ausbringens eine gezielte Dosierung möglich.

Der besondere Vorteil der vorgeschlagenen technischen Lösung liegt in der Möglichkeit, sowohl stationäre (at line aus Probenbehältern) als auch mobile (in line während des Ausbringens organischer Reststoffe) Bestimmungen von Bestandteilen und Konzentrationen in der Probe enthaltener Stoffe durchführen zu können.

Weiterhin ist die spektroskopische Messung nach dem Transflexionsprinzip besonders vorteilhaft. Dadurch ist es möglich, direkte Messung der Probe durch Lichtabsorption und/oder -transmission durchzuführen, ohne den Messaufbau verändern zu müssen. In Abhängigkeit von der zu messenden Probe, speziell der enthaltenen festen, flüssigen und suspensiven organischen Reststoffe, liefert die Messung transmissive und/oder reflexive Messergebnisse.

Der Vorgang der Säuberung, Spülung und Trocknung der Messzelle, um diese für die Messung anderer Proben vorzubereiten, kann automatisiert und von der Steuer- und Auswerteeinheit geregelt und kontrolliert werden. Der Vorgang der Säuberung, Spülung und Trocknung kann dabei spektroskopisch kontrolliert werden. In bezug auf vorhandene Referenzstandards kann vom Messkopf in Verbindung mit der Steuer- und Auswerteeinheit sowohl der Grad der Verschmutzung als auch der Feuchtigkeitsgrad der Messzelle ermittelt werden.

Nach Erkennung des Zustandes "Zelle trocken" erfolgt die Referenzierung durch automatischen Weiß/Schwarzabgleich des Spektrometers, mittels üblicher bekannter Referenzstandards. Eine derartige Referenzierung kann dabei nach Bedarf oder auch periodisch erfolgen. Ebenso ist realisierbar, dass die Überwachung des Verschmutzungsgrades der Messzelle durch das System selbst erfolgt.

Die erfindungsgemäße Lösung bietet auch die Möglichkeit einer SelbstKalibrierung. Nachdem von der Steuer- und Auswerteeinheit der Vorgang der Säuberung, Spülung und Trocknung geregelt und kontrolliert wird, kann über Mehrwegeventil beispielsweise auch ein oder mehrere Testflüssigkeiten definierter Zusammensetzung und bekannter Absorption bzw. Transmission in die Messzelle geleitet werden. Anhand dieser Kalibrationsflüssigkeit lässt sich das spektroskopische Signal kalibrieren und justieren. Dadurch kann eine gleichbleibend hohe Messgenauigkeit des Systems erreicht werden. Auch die hierbei verwendeten Testflüssigkeiten können nach dem Durchspülen der Messzelle **3** über das Mehrwegeventil **6** ausgestoßen werden.

Mit der erfindungsgemäßen Lösung können vollständige Teilproben oder komplette Chargen kontinuierlich gemessen werden, ohne dass die Proben vorbehandelt werden müssen. Die Proben werden durch den Messvorgang nicht verändert. Die Lösung kann auf eine Vielzahl relevanter Parameter kalibriert werden und liefert eine schnelle Analyse bei hoher Messdatendichte. Aufgrund der sehr kompakten und störunanfälligen Bauweise ist die Lösung insbesondere für mobile Einsätze geeignet.

Durch die schnelle und kontinuierliche Messwerterfassung ist die Lösung sowohl zur inline- als auch zur atline-Bestimmung der Inhaltsstoffe fester, flüssiger und/oder suspensiver organischer Reststoffe geeignet.

## Patentansprüche

1. Fahrzeug, auf dem eine Anordnung zur spektroskopischen Bestimmung der Bestandteile und Konzentrationen pumpfähiger Materialien montiert ist, umfassend einen Probenbehälter (1), eine Pumpe (2) und eine Messzelle (3), die mit einem spektroskopischen Messkopf (4) eine Einheit bildet, wobei die Messzelle (3) mit der Pumpe (2), die zur Variation der Fließgeschwindigkeit regelbar ist, und dem Probenbehälter (1) über eine Rohrleitung (5) verbunden ist und bei der der spektroskopische Messkopf (4) zu einer Steuer- und Auswerteeinheit elektrische Verbindungen aufweist, wobei die Durchflussmenge eines in der Auslassleitung eines Tankes des Fahrzeuges vorhandenen Auslassventils von der Steuer- und Auswerteeinheit geregelt wird.

2. Fahrzeug nach Anspruch 1, bei der die Messzelle (3) so ausgebildet ist, dass die Probe zwischen zwei gegenüberliegend, senkrecht zur Flussrichtung in die Messzelle (3) integrierten Fenstern, durchfließt.

3. Fahrzeug nach einem der Ansprüche 1 bis 2, bei der in der Rohrleitung (5) ein Mehrwegeventil (6) angeordnet ist, über welches Verbindungen zu einem Wasser- und/oder Reinigungsflüssigkeitsbehälter hergestellt werden können.

4. Fahrzeug nach einem der vorgenannten Ansprüche, bei der das in der Rohrleitung (5) angeordnete Mehrwegeventil (6) zur Selbstkalibrierung Verbindungen zu einem oder mehreren Behältern mit Testflüssigkeiten herstellen kann.

5. Fahrzeug nach Anspruch 3 oder 4, bei der das Mehrwegeventil (6) über einen Stellantrieb verfügt, der mit der Steuer- und Auswerteeinheit verbunden ist.

6. Fahrzeug nach einem der vorgenannten Ansprüche, bei dem eine Vorrichtung zur Trocknung der Messzelle (3) vorhanden ist, welche mit der Steuer- und Auswerteeinheit verbunden ist.

7. Fahrzeug nach einem der vorgenannten Ansprüche, bei dem eine Vorrichtung zur Temperierung der Probe vorhanden ist, welche mit der Steuer- und Auswerteeinheit verbunden ist.

8. Fahrzeug nach einem der vorgenannten Ansprüche, bei der die Anordnung über zwei Dreiwegeventile (8, 9) mit der Auslassleitung desauf dem Fahrzeug angeordneten Tanks verbunden ist.

9. Fahrzeug nach mindestens einem der vorgenannten Ansprüche, das zum Ausbringen pumpfähiger organischer Reststoffe vorgesehen ist.

10. Verfahren zur spektroskopischen Bestimmung der Bestandteile und Konzentrationen pumpfähiger Materialien, bei dem eine Probe von einer Pumpe (2) über eine Rohrleitung (5) durch eine Messzelle (3), die mit einem spektroskopischen Messkopf (4) eine Einheit bildet, gefördert wird, der Messkopf (4) eine spektroskopische Messung der durch die Messzelle (3) fließenden Probe in Transmission und/oder Reflexion vornimmt, und die Messergebnisse zur weiteren Verarbeitung an eine Steuer- und Auswerteeinheit übermittelt, von der anhand hinterlegter spezifischer Kalibrierungen Bestandteile und Konzentrationen in der Probe enthaltener Stoffe ermittelt werden, wobei die zu messende Probe über ein erstes in der Rohrleitung (5) angeordnete Dreiwegeventil (8) aus der Auslassleitung eines auf einem Fahrzeug angeordneten Tanks entnommen und nach erfolgter Messung über eine zweites in der Rohrleitung (5) angeordnete Dreiwegeventile (9) in die Auslassleitung zurückgefördert wird und wobei die Durchflussmenge des Auslassventils von der Steuer- und Auswerteeinheit geregelt wird.

11. Verfahren nach Anspruch 10, bei dem die Pumpe (2) regelbar ist, um die für die spektroskopische Messung erforderliche Fließgeschwindigkeit der Probe gewährleisten zu können.

12. Verfahren nach einem der Ansprüche 10 oder 11, bei dem ein vorhandener Wasserbehälter über ein Mehrwegeventil (6) mit der Messzelle (3) verbunden wird, um diese von Resten der gemessenen Probe zu säubern und für die nächste Probe vorzubereiten.

13. Verfahren nach Anspruch 10 oder 11, bei dem ein vorhandener Wasserbehälter und ein Behälter mit Reinigungsflüssigkeit über ein Mehrwegeventil (6) nacheinander mit der Messzelle (3) verbunden werden, um diese von Resten der gemessenen Probe zu säubern, zu spülen und für die nächste Probe vorzubereiten.

14. Verfahren nach einem der vorgenannten VerfahrensAnsprüche, bei dem die Messzelle (3) nach erfolgter Säuberung durch eine Vorrichtung zur Trocknung von Flüssigkeitsresten befreit wird.

15. Verfahren nach einem der vorgenannten VerfahrensAnsprüche, bei dem ein oder mehrere Behälter mit Testflüssigkeiten zur Selbstkalibrierung der Anordnung über ein Mehrwegeventil (6) mit der Messzelle (3) verbunden werden können.

16. Verfahren nach einem der vorgenannten VerfahrensAnsprüche, bei dem zur Vermeidung temperaturbeeinflusster Messergebnisse die Probe durch eine Vorrichtung temperiert werden kann.

17. Verfahren nach einem der vorgenannten VerfahrensAnsprüche, bei dem der Messkopf (4) eine spektroskopische Messung der Messzelle ohne eine Probe vornimmt, um den Verschmutzungsgrad der Messzelle (3) festzustellen.

18. Verfahren nach einem der vorgenannten VerfahrensAnsprüche, bei dem die Säuberung und/oder Trocknung der Messzelle (3), sowie eine mögliche Temperierung der Probe von der Steuer- und Auswerteeinheit gesteuert wird.

19. Verfahren nach einem der vorgenannten VerfahrensAnsprüche, bei dem von der Steuer- und Auswerteeinheit anhand der ermittelten Bestandteilen und Konzentrationen in der Probe enthaltener Stoffe ein zusätzliches Steuersignal zur Regelung des Durchflusses eines Auslassventils beim Ausbringen pumpfähiger organischer Reststoffe generiert wird.

20. Verfahren nach einem der vorgenannten VerfahrensAnsprüche, bei dem von der Steuer- und Auswerteeinheit zusätzlich zu den ermittelten Bestandteilen und Konzentrationen in der Probe enthaltener Stoffe zuvor bestimmte Bodenwerte sowie die Momentangeschwindigkeit des Fahrzeuges berücksichtigt werden, um ein Steuersignal zur Regelung des Durchflusses eines Auslassventils beim Ausbringen pumpfähiger organischer Reststoffe zu generieren.

## Claims

1. Vehicle, on which an arrangement for the spectroscopic determination of the constituents and concentrations of pumpable materials is mounted, including a sample container (1), a pump (2) and a measuring cell (3) which forms a unit with a spectroscopic measuring head (4), wherein the measuring cell (3) is connected to the pump (2), which is regulable for varying the flow rate, and to the sample container (1) by way of a pipeline (5), and in which the spectroscopic measuring head (4) has electrical connections with a control and evaluation unit, wherein the quantity of flow through an outlet valve in the outlet line of a tank of the vehicle is regulated by the control and evaluation unit.

2. Vehicle according to Claim 1, in which the measuring cell (3) is constructed such that the sample flows between two windows that are integrated in the measuring cell (3) such that they are opposite one another and perpendicular to the direction of flow.

3. Vehicle according to one of Claims 1 to 2, in which there is arranged in the pipeline (5) a multiway valve (6) by way of which connections can be made with a water and/or cleaning fluid container.

4. Vehicle according to one of the preceding claims, in which the multiway valve (6) arranged in the pipeline (5) can make connections with one or more containers with test fluids, for self-calibration.

5. Vehicle according to Claim 3 or 4, in which the multiway valve (6) has a servo drive which is connected to the control and evaluation unit.

6. Vehicle according to one of the preceding claims, in which a device for drying the measuring cell (3) is provided and is connected to the control and evaluation unit.

7. Vehicle according to one of the preceding claims, in which a device for controlling the temperature of the sample is provided and is connected to the control and evaluation unit.

8. Vehicle according to one of the preceding claims, in which the arrangement is connected by way of two three-way valves (8, 9) to the outlet line of the tank arranged on the vehicle.

9. Vehicle according to at least one of the preceding claims, which is provided for the output of pumpable organic residues.

10. Method for the spectroscopic determination of the constituents and concentrations of pumpable materials, in which a sample is conveyed by a pump (2), by way of a pipeline (5), through a measuring cell (3) that forms a unit with a spectroscopic measuring head (4), the measuring head (4) performs a spectroscopic measurement of the sample flowing through the measuring cell (3) in transmission and/or reflectance, and the measurement results are transmitted for further processing to a control and evaluation unit by which the constituents and concentrations of substances in the sample are determined with reference to specific stored calibrations, wherein the sample to be measured is taken, by way of a first three-way valve (8) arranged in the pipeline (5), from the outlet line of a tank arranged on a vehicle, and once measurement has been performed is conveyed back into the outlet line by way of a second three-way valve (9) arranged in the pipeline (5), and wherein the quantity of flow through the outlet valve is regulated by the control and evaluation unit.

11. Method according to Claim 10, in which the pump (2) is regulable so that the flow rate of the sample which is needed for the spectroscopic measurement can be ensured.

12. Method according to one of Claims 10 or 11, in which a water container that is provided is connected to the measuring cell (3) by way of a multiway valve (6) in order to clean the measuring cell (3) of residues from the measured sample and to prepare it for the next sample.

13. Method according to Claim 10 or 11, in which a water container that is provided and a container with cleaning fluid are successively connected to the measuring cell (3) by way of a multiway valve (6) in order to clean the measuring cell (3) of residues from the measured sample, to flush it and to prepare it for the next sample.

14. Method according to one of the preceding method claims, in which, once the cleaning has been performed, the measuring cell (3) is freed of residual fluid by a device for drying.

15. Method according to one of the preceding method claims, in which one or more containers with test fluids may be connected to the measuring cell (3) by way of a multiway valve (6), for self-calibration of the arrangement.

16. Method according to one of the preceding method claims, in which, for avoiding measurement results affected by temperature, the temperature of the sample can be controlled by a device.

17. Method according to one of the preceding method claims, in which the measuring head (4) performs a spectroscopic measurement of the measuring cell without a sample in order to determine the degree of soiling of the measuring cell (3).

18. Method according to one of the preceding method claims, in which cleaning and/or drying of the measuring cell (3), and possible control of the temperature of the sample, are controlled by the control and evaluation unit.

19. Method according to one of the preceding method claims, in which the control and evaluation unit generates, with reference to the determined constituents and concentrations of substances in the sample, an additional control signal for regulating the flow through an outlet valve during the output of pumpable organic residues.

20. Method according to one of the preceding method claims, in which the control and evaluation unit, in addition to the determined constituents and concentrations of substances in the sample, takes into account previously determined base values and the current speed of the vehicle in order to generate a control signal for regulating the flow through an outlet valve during the output of pumpable organic residues.

## Revendications

1. Véhicule sur lequel est monté un dispositif de détermination spectroscopique des constituants et des concentrations de matériaux aptes au pompage, comprenant un récipient à échantillons (1), une pompe (2) et une cellule de mesure (3) formant une unité avec une tête de mesure spectroscopique (4), dans lequel la cellule de mesure (3) est reliée à la pompe (2), qui peut être réglée pour faire varier la vitesse d'écoulement, et au récipient à échantillon (1) par l'intermédiaire d'une conduite (5) et dans lequel la tête de mesure spectroscopique (4) présente des connexions électriques vers une unité de commande et d'évaluation, dans lequel le débit d'une soupape d'échappement présente dans la conduite d'échappement d'un réservoir du véhicule est réglé par l'unité de commande et d'évaluation.

2. Véhicule selon la revendication 1, dans lequel la cellule de mesure (3) est conçue de manière à ce que l'échantillon s'écoule entre deux fenêtres intégrées à la cellule de mesure (3) et opposées l'une à l'autre, perpendiculairement à la direction d'écoulement.

3. Véhicule selon l'une quelconque des revendications 1 à 2, dans lequel une soupape à plusieurs voies (6) est disposée dans la conduite (5), soupape par l'intermédiaire de laquelle des liaisons peuvent être établies vers un récipient d'eau et/ou de liquide de nettoyage.

4. Véhicule selon l'une quelconque des revendications précédentes, dans lequel la soupape à plusieurs voies (6) disposée dans la conduite (5) peut établir des liaisons vers un ou plusieurs récipients contenant des liquides de test à des fins d'auto-étalonnage.

5. Véhicule selon l'une quelconque des revendications 3 ou 4, dans lequel la soupape à plusieurs voies (6) comporte un mécanisme de réglage qui est relié à l'unité de commande et d'évaluation.

6. Véhicule selon l'une quelconque des revendications précédentes, dans lequel il est prévu, pour sécher la cellule de mesure (3), un dispositif qui est relié à l'unité de commande et d'évaluation.

7. Véhicule selon l'une quelconque des revendications précédentes, dans lequel il est prévu un dispositif de régulation thermique des échantillons, qui est relié à l'unité de commande et d'évaluation.

8. Véhicule selon l'une quelconque des revendications précédentes, dans lequel le dispositif est relié par l'intermédiaire de deux soupapes à trois voies (8, 9) à la conduite de sortie du réservoir disposé sur le véhicule.

9. Véhicule selon au moins l'une des revendications précédentes, qui est prévu pour expulser des résidus organiques aptes au pompage.

10. Procédé de détermination spectroscopique des constituants et des concentrations de matériaux aptes au pompage, dans lequel un échantillon est entraîné d'une pompe (2) par l'intermédiaire d'une conduite (5), à travers une cellule de mesure (3) qui forme une unité avec une tête de mesure spectroscopique (4), la tête de mesure (4) effectue une mesure en transmission et/ou en réflexion de l'échantillon s'écoulant à travers la cellule de mesure (3) et les résultats de mesure sont transmis en vue d'un traitement supplémentaire à une unité de commande et d'évaluation, à partir desquels, sur la base d'étalonnages spécifiques stockés, des constituants et des concentrations en matériaux contenus dans l'échantillon sont déterminés, dans lequel l'échantillon à mesurer est prélevé par l'intermédiaire d'une première soupape à trois voies (8) disposée dans la conduite (5) à partir de la conduite de sortie d'un réservoir disposé sur un véhicule et, après que la mesure a été effectuée, est renvoyé par l'intermédiaire d'une seconde soupape à trois voies (9) disposée dans la conduite (5) dans la conduite d'échappement et dans lequel le débit de la soupape d'échappement est réglé par l'unité de commande et d'évaluation.

11. Procédé selon la revendication 10, dans lequel la pompe (2) peut être réglée afin de pouvoir garantir la vitesse d'écoulement de l'échantillon qui est nécessaire pour la mesure spectroscopique.

12. Procédé selon l'une quelconque des revendications 10 ou 11, dans lequel un récipient d'eau présent est relié par l'intermédiaire d'une soupape à plusieurs voies (6) à la cellule de mesure (3) afin d'éliminer les résidus de l'échantillon mesuré et d'effectuer la préparation à l'échantillon suivant.

13. Procédé selon la revendication 10 ou 11, dans lequel un récipient d'eau présent et un récipient contenant un liquide de nettoyage sont reliés l'un après l'autre à la cellule de mesure (3) par l'intermédiaire d'une soupape à plusieurs voies (6) afin d'éliminer de celle-ci des résidus de l'échantillon mesuré à des fins de rinçage et de préparation à l'échantillon suivant.

14. Procédé selon l'une quelconque des revendications précédentes, dans lequel la cellule de mesure (3) est libérée, une fois le nettoyage effectué, par un dispositif destiné à sécher les résidus de liquide.

15. Procédé selon l'une quelconque des revendications précédentes, dans lequel un ou plusieurs récipients contenant des liquides de test destinés à l'auto-étalonnage du dispositif peuvent être reliés à la cellule de mesure (3) par l'intermédiaire d'une soupape à plusieurs voies (6).

16. Procédé selon l'une quelconque des revendications précédentes, dans lequel l'échantillon peut être soumis à une régulation de température au moyen d'un dispositif afin d'éviter l'obtention de résultats de mesure influencés par la température.

17. Procédé selon l'une quelconque des revendications précédentes, dans lequel la tête de mesure (4) effectue une mesure spectroscopique de la cellule de mesure sans échantillon afin d'établir le degré de salissure de la cellule de mesure (3).

18. Procédé selon l'une quelconque des revendications précédentes, dans lequel le nettoyage et/ou le séchage de la cellule de mesure (3) et une régulation de température possible de l'échantillon sont commandés par l'unité de commande et d'évaluation.

19. Procédé selon l'une quelconque des revendications précédentes, dans lequel un signal de commande supplémentaire destiné à régler le débit d'une soupape d'échappement lors de l'expulsion de matériaux résiduels organiques aptes au pompage est généré par l'unité de commande et d'évaluation sur la base des constituants et des concentrations déterminés de composés contenus dans l'échantillon.

20. Procédé selon l'une quelconque des revendications de procédé précédentes, dans lequel, en plus des constituants et des concentrations de composés contenus dans l'échantillon, des valeurs de base déterminées au préalable et la vitesse instantanée du véhicule sont prises en compte par l'unité de commande et d'évaluation afin de générer un signal de commande destiné à régler le débit d'une soupape d'échappement lors de l'expulsion de composés résiduels organiques aptes au pompage.
